# EUROPÄISCHE PATENTSCHRIFT

(11) **EP 1 657 243 B1**
(45) Veröffentlichungstag und Bekanntmachung des Hinweises auf die Patenterteilung: **18.08.2010**
(21) Anmeldenummer: 06002571.5
(22) Anmeldetag: 24.11.1998
(51) Int. Cl.: C07D 473/00, C07C 213/02, C07C 215/42, C07C 271/24, C07C 229/48

(54) **Verfahren zur Herstellung von Aminoalkoholen**
Procedure for the production of amino alcohols
Procédure pour la production des alcools aminés

(30) Priorität: 27.11.1997 CH 273997; 03.12.1997 CH 278197; 21.01.1998 CH 13398; 27.03.1998 CH 72398; 07.10.1998 EP 98118895
(43) Veröffentlichungstag der Anmeldung: 17.05.2006
(62) Teilanmeldung aus: 04002913.4
(73) Patentinhaber: Lonza AG, 3930 Visp (CH)
(72) Erfinder: Berchtold, Katja, 3937 Baltschieder (CH); Roduit, Jean-Paul, 1950 Sion (CH); Bernegger-Egli, Christine, Dr., 3985 Münster (CH); Urban, Eva Maria, 3912 Termen (CH); Petersen, Michael, Dr., 79540 Lörrach (DE)
(74) Vertreter: Winter, Brandl, Fürniss, Hübner, Röss, Kaiser, Polte - Partnerschaft

(56) Entgegenhaltungen:
- WO-A-93/17020
- WO-A-95/21161
- CAMPBELL J A ET AL: "CHIROSPECIFIC SYNTHESES OF PREDURSORS OF CYCLOPENTANE AND CYCLOPENTENE CARBOCYCLIC NUCLEOSIDES BY Ú3 + 3-COUPLING AND TRANSANNULAR ALKYLATION" JOURNAL OF ORGANIC CHEMISTRY, AMERICAN CHEMICAL SOCIETY. EASTON, US, Bd. 60, Nr. 14, 1995, Seiten 4602-4616, XP002041314 ISSN: 0022-3263
- TANAKA, MASAKAZU ET AL: "Chemoenzymatic Synthesis of Antiviral Carbocyclic Nucleosides: Asymmetric Hydrolysis of meso-3,5-Bis(acetoxymethyl)cyclopentenes Using Rhizopus delemar Lipase" JOURNAL OF ORGANIC CHEMISTRY, Bd. 61, 1996, Seiten 6952-6957, XP002372987
- KATAGIRI N ET AL: "Deamination of 9-(hydroxymethylated cycloalkyl)-9H-adenines (carbocyclic adenine nucleosides) by adenosine deaminase: Effect of the high-pressure upon deamination rate and enantioselectivity" NUCLEOSIDES & NUCLEOTIDES, MARCEL DEKKER, INC, US, Bd. 15, Nr. 1/3, 1996, Seiten 631-647, XP002124310 ISSN: 0732-8311

## Beschreibung

Die vorliegende Erfindung betrifft ein Verfahren zur Herstellung eines Aminoalkohols der Formel 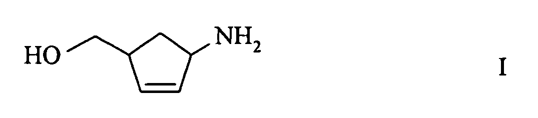 in Form des Racemats oder der optisch aktiven Isomeren.

(1R,4S)-1-Amino-4-(hydroxymethyl)-2-cyclopenten ist ein wichtiges Zwischenprodukt zur Herstellung von carbocyclischen Nukleosiden wie z. B. Carbovir^{®} (Campbell et al., J. Org. Chem. 1995, 60, 4602 - 4616).

Ein Verfahren zur Herstellung von (1R,4S)-1-Amino-4-(hydroxymethyl)2-cyclopenten wird beispielsweise von Campbell et al. (J. Org. Chem. 1995, 60, 4602-4616) beschrieben. Die Herstellung erfolgt durch Reduktion und Hydrolyse bicyclischer Carbamate unter Abspaltung einer tert-Butoxycarbonyl-Schutzgruppe und Ringöffnung. Ein anderes Verfahren wird zur Herstellung von (1R,4S)-1-Amino-4-(hydroxymethyl)-2-cyclopenten wird bei Park K. H. & Rapoport H. (J. Org. Chem. 1994, 59, 394 - 399) beschrieben.Beide Verfahren sind kostspielig, umständlich und grosstechnisch nicht gangbar.

Die WO 93/17020 beschreibt ein Verfahren zur Herstellung von (1R,4S)-1-Amino-4-(hydroxymethyl)-2-cyclopenten, wobei (1R,4S)-4-Amino-2-cyclopenten-1-carbonsäure mit Lithiumaluminiumhydrid ins gewünschte Produkt reduziert wird. Nachteilig bei diesem Verfahren ist zum einen, dass auch die Doppelbindung des Cyclopentenringes reduziert wird, die schlechte Handhabbarkeit von Lithiumaluminiumhydrid und zum anderen, dass es zu kostspielig ist.

Tanaka, M. et al. (J. Org. Chem. 1996, 61, 6952-6957) beschreiben die Bildung eines 1-Amino-4-hydroxymethyl-2-cyclopentens durch Erhitzen aus eines Alkylcarbamats, Hydroxymethyl-3-[(methoxycarbonyl)amino]cyclopenten, mit Kalilauge.

Ähnlich beschreiben Katagiri, N. et al. (Nucleosides & Nucleotides, 1996, 15, 631-647) die Herstellung von 1-Amino-4-hydroxymethyl-2-cyclopenten durch Behandeln eines Carbamats mit Trifluoressigsäure.

Taylor S. J. et al. (Tetrahetron: Asymmetry Vol. 4, No. 6, 1993, 1117 - 1128) beschreiben ein Verfahren zur Herstellung von (1R,4S)-1-Amino-4-(hydroxymethyl)-2-cyclopenten ausgehend von (±)-2-Azabicyclo[2.2.1]hept-5-en-3-on als Edukt. Dabei wird das Edukt mittels Mikroorganismen der Spezies Pseudomonas solanacearum oder Pseudomonas fluorescens in (1R,4S)-2-Azabicyclo[2.2.1]hept-5-en-3-on überführt, welches dann mit Di-tert-butyldicarbonat in das (1R,4S)-N-tert-butoxycarbonyl-2-azabicyclo[2.2.1]hept-5-en-3-on umgesetzt wird und letzteres mit Natriumborhydrid und Trifluoressigsäure ins gewünschte Produkt reduziert wird. Dieses Verfahren ist viel zu kostspielig.

Desweiteren beschreiben Martinez et al. (J. Org. Chem. 1996, 61, 7963 - 7966) eine 10stufige Synthese von (1R,4S)-1-Amino-4-(hydroxymethyl)-2-cyclopenten ausgehend von Dialkylmalonsäurediethylester. Auch dieses Verfahren hat den Nachteil, dass es umständlich und grosstechnisch nicht gangbar ist.

Bekannt ist auch, dass man N-substituierte-(±)-2-Azabicyclo[2.2.1]hept-5-en-3-one, welche einen elektronenanziehenden Substituenten tragen mit einem Metallhydrid zu den entsprechenden N-substituierten Aminoalkoholen reduzieren kann (Katagiri et al., Tetrahedron Letters, 1989, 30, 1645 - 1648; Taylor et al., ibid).

Im Gegensatz dazu ist bekannt, dass unsubstituiertes (±)-2-Azabicyclo[2.2.1]hept-5-en-3-on der Formel 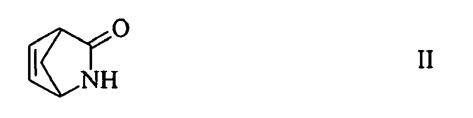 mit Lithiumaluminiumhydrid zu (±)-2-Azabicyclo[2.2.1]octen reduziert wird (Malpass & Tweedle, J. Chem. Soc., Perkin Trans 1, 1977, 874 - 884) und dass die direkte Reduktion von (±)-2-Azabicyclo[2.2.2]hept-5-en-3-on zum entsprechenden Aminoalkohol bislang als nicht möglich galt (Katagiri et al., Tetrahedron Letters, 1989, 30, 1645-1648; Taylor et al., ibid).

Bekannt ist auch, racemisches 1-Amino-4-(hydroxymethyl)-2-cyclopenten mittels (-)-Dibenzoylweinsäure zu spalten (US-A 5 034 394). Diese Umsetzung hat einerseits den Nachteil, dass die (-)-Dibenzoylweinsäure teuer ist, anderseits, dass die Trennung in Gegenwart eines genau definierten Gemisches von Acetonitril und Ethanol erfolgen muss. Dieses Lösungsmittelgemisch kann nicht getrennt werden und muss der Verbrennung zugeführt werden.

Aufgabe der vorliegenden Erfindung war es, ein einfaches, ökonomisches und kostengünstiges Verfahren zur Herstellung von 1-Amino-4-(hydroxymethyl)-2-cyclopenten zur Verfügung zu stellen.

Überraschend wurde gefunden, dass wenn man ein Cyclopentenderivat der allgemeinen Formel 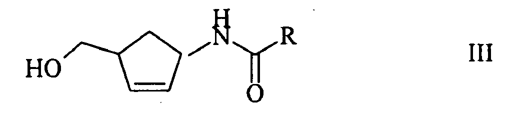 in Form des Racemats oder eines seiner optisch aktiven Isomere, worin R C₁₋₄-Alkyl oder Aryl bedeutet, mit einem Alkalimetallhydroxid hydrolysiert, der Aminoalkohol der Formel 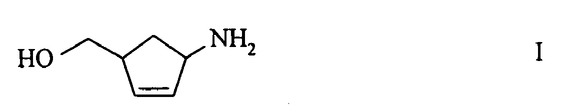 in Form des Racemats oder eines seiner optisch aktiven Isomere auf einfache Weise erhalten wird.

C₁₋₄-Alkyl kann substituiert oder unsubstituiert sein. Unter substituiertem C₁₋₄-Alkyl wird im folgenden mit einem oder mehreren Halogenatomen substituiertes C₁₋₄-Alkyl verstanden. Als Halogenatom kann F, Cl, Br oder J angewendet werden. Beispiele für C₁₋₄-Alkyl sind Methyl, Ethyl, Propyl, Butyl, Isobutyl, tert. Butyl, Isopropyl, Chlormethyl, Brommethyl, Dichlormethyl, Dibrommethyl. Vorzugsweise wird als C₁₋₄-Alkyl Methyl, Ethyl, Propyl, Butyl, Isobutyl oder Chlormethyl verwendet.

Als Aryl kann beispielsweise Phenyl oder Benzyl, substituiert oder unsubstituiert, verwendet werden. Als substituiertes Phenyl oder Benzyl wird im folgenden mit einem oder mehreren Halogenatomen substituiertes Phenyl oder Benzyl verstanden, wie beispielsweise Chlorbenzyl, Dichlorbenzyl, Bromphenyl oder Dibromphenyl.

Als Alkalimetallhydroxid kann Natrium- oder Kaliumhydroxid eingesetzt werden.

Der Aminoalkohol der Formel I kann mit einer Säure in die entsprechenden Salze überführt werden, wie beispielsweise in Hydrohalogenidsalze. Als Hydrohalogenidsalze sind Hydrobromide und Hydrochloride geeignet.

Das Cyclopentenderivat der allgemeinen Formel III wird bevorzugt durch Reduktion des entsprechenden Acyl-2-azabicyclo[2.2.1]-hept-5-en-3-ons der allgemeinen Formel 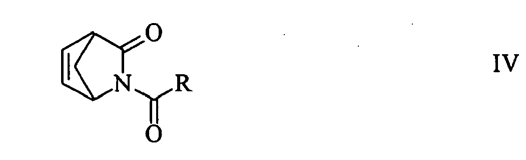 in Form des Racemats oder eines seiner optisch aktiven Isomere, worin R die genannte Bedeutung hat, mit einem Metallhydrid in einem wasserfreien Lösungsmittel hergestellt.

Als wasserfreies Lösungsmittel können protische oder aprotische organische Lösungsmittel eingesetzt werden, insbesondere wasserfreie protische organische Lösungsmittel wie ein tertiärer Alkohol. Als tertiäre Alkohole können tert-Butylalkohol und tert-Amylalkohol verwendet werden.

Als Metallhydride können Alkalimetall- oder Erdalkalimetallhydride sowie binäre oder komplexe Metallhydride der Bor- oder Aluminiumgruppe eingesetzt werden, wie Alkalimetall- und Erdalkalimetallborhydride und Alkalimetall- und Erdalkalimetallaluminiumhydride. Als Alkalimetall- oder Erdalkalimetallhydride sind LiH, NaH, KH, BeH₂, MgH₂ oder CaH₂ geeignet. Als binäre Alkalimetall- oder Erdalkalimetallborhydride können NaBH₄, LiBH₄, KBH₄, NaAlH₄, LiAlH₄, KalH₄, Mg(BH₄)₂, Ca(BH₄)₂, Mg(AlH₄)₂, Ca(AlH₄)₂ verwendet werden. Komplexe Metallhydride der Bor- oder Aluminiumgruppe können die allgemeine Formel M¹ M² HₙLₘ haben, worin n eine ganze Zahl von 1 bis 4 ist und m eine ganze Zahl von 4 bis 4 minus der entsprechenden Zahl n ist, M¹ ein Alkalimetallatom, M² Bor oder Aluminium bedeutet und L C₁₋₄-Alkyl, C₁₋₄-Alkenyl, C₁₋₄-Alkoxy, CN oder ein Amin ist, oder die komplexen Metallhydride können die allgemeine Formel M²H_{O}Lₚ haben, worin M² die genannte Bedeutung hat und O eine ganze Zahl von 0 bis 3 ist und p eine ganze Zahl von 3 bis 3 minus der entsprechenden Zahl p ist. Als M¹ M² HₙLₘ können LiBH (C₂H₅)₃, LiBHₓ (OCH₃)₄₋ₓ, worin x eine ganze Zahl von 1 bis 3 bedeutet, LiAlH(OC(CH₃)₃)₃, NaAlH₂(OC₂H₄OCH₃)₂, NaAlH₂(C₂H₅)₂ oder NaBH₃CN eingesetzt werden. Vorzugsweise wird die Reduktion mit einem Metallborhydrid durchgeführt.

Wie fachmännisch bekannt, können die erwähnten Metallhydride wie z. B. LiBH₄ auch "in-situ" erzeugt werden. Gängige Darstellungsmethoden für LiBH₄ sind beispielsweise die Umsetzung eines Alkalimetallborhydrids mit einem Lithiumhalogenid (H. C. Brown et al., Inorg. Chem. 20, 1981, 4456 - 4457), die Umsetzung von LiH mit B₂O₃ in Gegenwart von Wasserstoff und einem Hydrierkatalysator (EP-A 0 512 895), die Umsetzung von LiH mit (H₅C₂)OBF₃ (DE-OS 94 77 02) und die von LiH mit B(OCH₃)₃ (US-A 2,534,533).

Die Reduktion erfolgt bevorzugt in Gegenwart eines Zusatzes, wie in Gegenwart eines C₁₋₆-Alkohols wie Methanol, insbesondere in Gegenwart von 2 mol Methanol pro mol Acyl-2-Azabicyclo[2.2.1]-hept-5-en-3-on der Formel IV.

Zweckmässig wird die Umsetzung bei einer Temperatur von 0 bis 50 °C, vorzugsweise von 15 bis 30 °C durchgeführt.

Der so erhaltene racemische, bevorzugt der cis-racemische, Aminoalkohol der Formel I kann einer Racematspaltung unterzogen werden, beispielsweise biotechnologisch mit einer Hydrolase in Gegenwart eines Acylierungsmittels oder chemisch mit einer optisch aktiven Weinsäure wie D-(-)-Weinsäure oder L-(+)-Weinsäure.

### Beispiele:

### Beispiel 1

### Reduktion von Acyl-unsubstituiertem 2-Azabicyclo[2.2.1]hept-5-en-3-on

### Herstellung von cis-(±)-Acetyl-1-Amino-4-(hydroxymethyl)-2-cyclopenten in wasserfreiem protischen organischen Lösungsmittel mittels Natriumborhydrid

280 g 2-Methyl-2-butanol (Amylalkohol) und 15,2 g Natriumborhydrid (0,4 mol) wurden in einem Sulfierkolben bei 20 °C vorgelegt. Zu dieser Suspension wurde ein Gemisch aus 907 g (±)-Acetyl-2-Azabicyclo[2.2.1]hept-5-en-3-on (0,6 mol) und 37,5 g Methanol (2 Äquivalente bezogen auf (±)-Acetyl-2-Azabicyclo[2.2.1]hept-5-en-3-on) innert 2 h bei 20 °C zudosiert. Anschliessend wurde das Reaktionsgemisch noch 3 h bei 20°C gerührt. Das Lösungsmittel wurde soweit wie möglich abdestilliert (40 °C). Zur Entfernung von Bor wurden 280 g Methanol und 27,2 g Ameisensäure zugegeben, das Gemisch wurde auf 25-30 °C erwärmt und das Azeotrop Methylborat / Methanol wurde bei dieser Temperatur abdestilliert (130 bis 80 mbar). Das ausgefallene Natriumformiat wurde abfiltriert und das Filtrat eingeengt. Man erhielt 93,4 g Rohprodukt als klares viskoses Oel, Rohausbeute mit Gehalt: ca 84-85 %.

### Beispiel 2

### Alkalische Hydrolyse von Acetyl-(±)-1-Amino-4-(hydroxymethyl)-2-cyclopenten

88,9 g racemisches Acetyl-1-amino-4-(hydroxymethyl)-2-cyclopenten (Gehalt 77,2 %) wurden suspendiert (teilweise gelöst) in 70 g Wasser. Dazu wurde 84 g 30 % NaOH (1,1 Äquivalente) gegeben, die Lösung wurde 3 h rückflussiert. Laut DC war die Hydrolyse vollständig. Das entstandene Acetat wurde durch Elektrodialyse entfernt. Die erhaltene wässrige Lösung wurde eingeengt und getrocknet durch azeotrope Destillation mit Butanol. Der Rückstand wurde zur Racematspaltung in Methanol aufgenommen. Die Ausbeute der Hydrolyse zum (±)-1-Amino-4-(hydroxymethyl)-2-cyclopenten betrug 90 %.

## Patentansprüche

1. Verfahren zur Herstellung eines Aminoalkohols der Formel 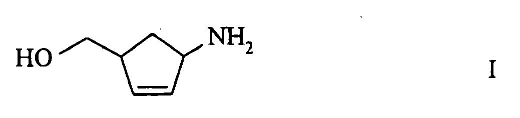 in Form des Racemats oder eines seiner optisch aktiven Isomeren, umfassend die Hydrolyse eines Cyclopentenderivats der allgemeinen Formel 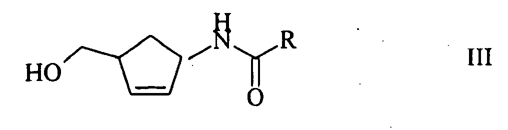 in Form des Racemats oder eines seiner optisch aktiven Isomeren, worin R C₁₋₄-Alkyl oder Aryl bedeutet, mit einem Alkalimetallhydroxid.

2. Verfahren nach Anspruch 1, **dadurch gekennzeichnet, dass** das Cyclopentenderivat der allgemeinen Formel 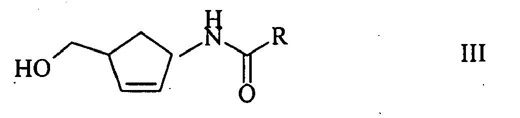 in Form des Racemats oder eines seiner optisch aktiven Isomeren, worin R die genannte Bedeutung hat, **dadurch** hergestellt wird, dass man ein Acyl-2-azabicyclo[2.2.1]hept-5-en-3-on der Formel 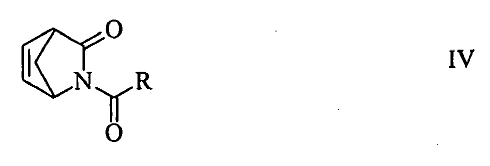 in Form des Racemats oder eines seiner optisch aktiven Isomeren, worin R die genannte Bedeutung hat, mit einem Metallhydrid in einem wasserfreien Lösungsmittel reduziert.

## Claims

1. Process for the preparation of an aminoalcohol of the formula 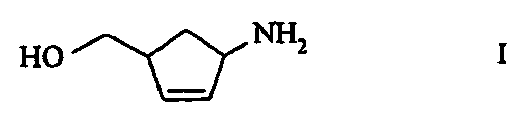 in the form of the racemate or one of its optically active isomers, comprising the hydrolysis of a cyclopentene derivative of the general formula 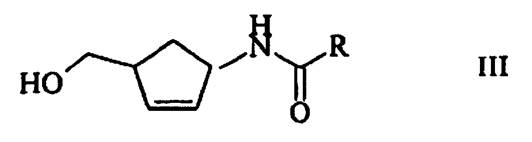 in the form of the racemate or one of its optically active isomers, in which R is C₁₋₄-alkyl or aryl, with an alkali metal hydroxide.

2. Process according to Claim 1, **characterized in that** the cyclopentene derivative of the general formula 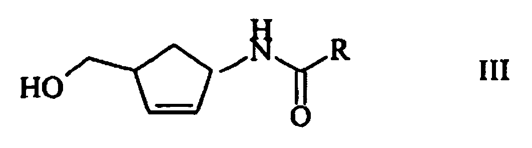 in the form of the racemate or one of its optically active isomers, in which R is as defined above, is prepared by reducing an acyl-2-azabicyclo[2.2.1]hept-5-en-3-one of the formula 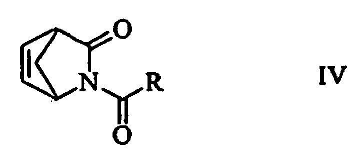 in the form of the racemate or one of its optically active isomers, in which R is as defined above, with a metal hydride in an anhydrous solvent.

## Revendications

1. Procédé pour la préparation d'un aminoalcool de formule 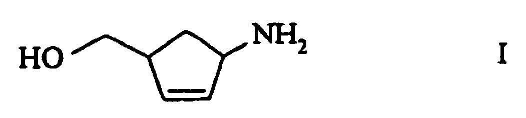 sous forme du racémate ou d'un de ses isomères optiquement actifs, comprenant l'hydrolyse d'un dérivé de cyclopentène de formule générale 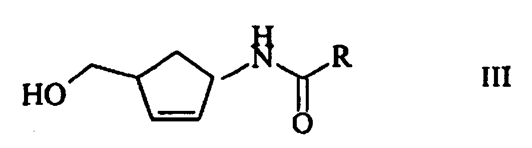 sous forme du racémate ou d'un de ses isomères optiquement actifs, où R signifie C₁₋₄-alkyle ou aryle, avec un hydroxyde de métal alcalin.

2. Procédé selon la revendication 1, **caractérisé en ce que** le dérivé de cyclopentène de formule générale 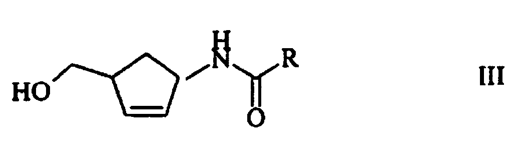 sous forme du racémate ou d'un de ses isomères optiquement actifs, où R a la signification susmentionnée, est préparé **en ce qu'**on réduit un acyl-2-azabicyclo[2,2,1]hept-5-én-3-one de formule 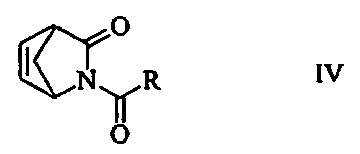 sous forme du racémate ou d'un de ses isomères optiquement actifs, où R présente la signification mentionnée, avec un hydrure de métal dans un solvant anhydre.
